Europäisches Patentamt

European Patent Office

Office européen des brevets

(11) Veröffentlichungsnummer : **0 027 501**
**B1**

(12) # EUROPÄISCHE PATENTSCHRIFT

(45) Veröffentlichungstag der Patentschrift :
22.09.82

(51) Int. Cl.³ : **C 07 C 69/16, C 07 C 67/36**

(21) Anmeldenummer : **80104290.4**

(22) Anmeldetag : **22.07.80**

(54) **Verfahren zur Herstellung von 1,1-Diacetoxiethan.**

(30) Priorität : **11.10.79 DE 2941232**

(43) Veröffentlichungstag der Anmeldung :
**29.04.81 (Patentblatt 81/17)**

(45) Bekanntmachung des Hinweises auf die Patenterteilung : **22.09.82 Patentblatt 82/38**

(84) Benannte Vertragsstaaten :
**AT BE CH DE FR GB IT LI NL**

(56) Entgegenhaltungen :
**DE A 2 610 035**
**DE A 2 733 663**
**DE C 313 696**

(73) Patentinhaber : **HOECHST AKTIENGESELLSCHAFT**
**Postfach 80 03 20**
**D-6230 Frankfurt/Main 80 (DE)**

(72) Erfinder : **Kübbeler, Hans-Klaus, Dr.**
**Bünnagelring 31**
**D-5357 Swisttal 8 (DE)**
Erfinder : **Erpenbach, Heinz, Dr.**
**Oberbuschweg 22**
**D-5000 Köln 50 (DE)**
Erfinder : **Gehrmann, Klaus, Dr.**
**Geschwister-Scholl-Strasse 32**
**D-5042 Erftstadt (DE)**
Erfinder : **Kohl, Georg**
**von Geyr-Ring 61a**
**D-5030 Hürth (DE)**

Imprimerie Jouve, 18, rue St-Denis, 75001 Paris, France

## Verfahren zur Herstellung von 1,1-Diacetoxiethan

Die Erfindung betrifft ein Verfahren zur Herstellung von 1-1-Diacetoxiethan (Ethylidendiacetat) durch Umsetzung von Methylacetat und/oder Dimethylether mit Kohlenmonoxid und Wasserstoff unter praktisch wasserfreien Bedingungen bei Temperaturen von 350 bis 575 K und Drücken von 1 bis 300 bar in Gegenwart eines Katalysatorsystems, welches Edelmetalle der Gruppe VIII des Periodensystems der Elemente oder deren Verbindungen und Jod und/oder dessen Verbindungen enthält, und ist dadurch gekennzeichnet, daß man in Gegenwart eines Katalysatorsystems arbeitet, welches zusätzlich eine aliphatische Carbonsäure mit 1-8 Kohlenstoffatomen, eine heterocyclische aromatische Verbindung, in der mindestens ein Heteroatom ein quaternäres Stickstoffatom ist und deren Schmelz- oder Mischschmelzpunkt unterhalb 410 K liegt, sowie eine Verbindung des Mangans oder Rheniums enthält.

Die DE-A1-26 10 035 beschreibt bereits ein vergleichbares Verfahren zur Herstellung von 1,1-Diacetoxiethan (Ethylidendiacetat), jedoch mit dem Unterschied, daß neben dem Edelmetall der Gruppe VIII des Periodensystems und einem Jodid ein Mehrfachpromotor eingesetzt wird, der mindestens einen Vertreter der Chrom, Cobalt, Eisen und Nickel umfassenden Gruppe sowie eine Organophosphin-, Organoarsin-, Organostibin-, Organostickstoff- und/oder Organosauerstoff-Verbindung enthält.

Die DE-C-313 696 beschreibt u.a. die Herstellung von Ethylidendiacetat durch Umsetzung von Vinylacetat und Essigsäure in Gegenwart von z.B. konzentrierter Schwefelsäure als Katalysator unter Kochen am Rückfluß ohne Angabe einer Ausbeute.

Die DE-A1-27 33 663 betrifft u.a. die Umsetzung von Dimethylether mit Kohlenmonoxid und Wasserstoff in Gegenwart von Rutheniumcarbonyl und einer Jodverbindung (z.B. $CH_3I$), wobei neben Essigsäuremethylester auch Essigsäureethylester, Methan, niedere Alkanole, Carbonsäuren und höhere Ether, jedoch weder Ethylidendiacetat noch Acetanhydrid gebildet werden.

Beim Verfahren der DE-A1-26 10 035 wirkt es sehr es sehr störend, daß die Metallverbindungen und Folgeprodukte des Mehrfachpromotors in 1,1-Diacetoxiethan weitgehend unlöslich sind, so daß die beim kontinuierlichen Verfahren erforderliche Kreislaufführung des Katalysatorsystems sehr erschwert, wenn nicht gar unmöglich gemacht wird. Ebenso beeinträchtigen diese unlöslichen Verbindungen die Trennung des 1,1-Diacetoxiethans vom Katalysatorsystem in unzulässigem Maße. Als Folge davon müßte eine langwierige, verlustreiche Zwischenbehandlung des teuren, edelmetallhaltigen Katalysators vorgenommen werden, wobei die auftretenden Katalysatorverluste zu einer schnellen Aktivitätsminderung im gesamten System führen. Diese Umstände verhinderten bisher die Durchführung des Verfahrens im technischen Maßstab. Darüberhinaus bewirkt gemäß den entsprechenden Beispielen der DE-A1-26 10 035 und nachfolgendem Beispiel 2 ein Zusatz des vorzugsweise als Promotor benutzten Chroms unter vergleichbaren Bedingungen eine deutliche Verschlechterung der Katalysatorleistung hinsichtlich 1,1-Diacetoxiethan zugunsten von Essigsäureanhydrid.

Die eingangs geschilderte Erfindung bewirkt nun eigenartigerweise eine bedeutende Steigerung der Katalysatoraktivität, ausgedrückt in Gramm erhaltenen 1,1-Diacetoxiethans und Essigsäureanhydrids je Gramm Edelmetall und Stunde, wobei die Selektivität der Bildung von 1,1-Diacetoxiethan im wesentlichen unverändert bleibt.

Diese Beobachtung ist um so überraschender, als die Löslichkeit von Verbindungen des Mangans und Rheniums im Reaktionssystem nicht vorauszusehen war und aus der Literatur Beispiele für eine derartige aktivitätserhöhende Wirkung der Verbindungen des Mangans und Rheniums bei der hydrierenden Carbonylierung von Estern und/oder Ethern nicht bekannt sind. Derartige, im Rahmen der Erfindung verwendbare Verbindungen des Mangans und Rheniums sind zum Beispiel Manganacetat, Manganchlorid, Manganjodid, Mangancarbonyl, Cyclopentadienylmangantricarbonyl, Rheniumchlorid, Rheniumoxichlorid, Rheniumdecacarbonyl.

Die vorliegende Erfindung vermeidet weiterhin die für die DE-A1-26 10 035 aufgeführten Nachteile, denn die erfindungsgemäß eingesetzten heterocyclischen aromatischen Verbindungen mit mindestens einem quaternären Stickstoffatom als Heteroatom bilden einzeln oder im Gemisch sowohl unter Reaktionsbedingungen als auch unter den Bedingungen der Aufarbeitung der Reaktionsprodukte der hydrierenden Carbonylierung von Methylacetat bzw. Dimethylether Schmelzen, die sich als geeignete Lösemittel für die Edelmetallkomplexe und die Verbindungen des Mangans oder Rheniums erweisen und darüber hinaus mit dem 1,1-Diacetoxyethan gut mischbar sind.

Die Erfindung ist bevorzugt und wahlweise dadurch gekennzeichnet, daß man

a) die heterocyclischen Verbindungen in Form ihrer Addukte mit Eissigsäure oder Methyljodid einsetzt;

b) das Katalysatorsystem Edelmetall(-verbindung)/Mangan- oder Rheniumverbindung/Jod(-verbindung)/Carbonsäure/heterocyclische Verbindung im Atom- bzw. Molverhältnis 1 : (0,1-10) : (1-1 400) : (10-2 000) : (1-1 200) einsetzt;

c) Kohlenmonoxid/Wasserstoffgelmische mit 25 bis 75 Volum % Wasserstoff einsetzt.

Als heterocyclische aromatische Verbindungen mit den geforderten Eigenschaften kommen erfindungsgemäß beispielsweise infrage:

1. N-Methylpyridiniumjodid, N,N-Dimethylimidazoliumjodid, N-Methyl-3-picoliniumjodid, N-Methyl-2,4-lutidiniumjodid, N-Methyl-3,4-lutidiniumjodid, N-Methyl-chinoliniumjodid ;

2. Pyridiniumacetat, N-Methylimidazoliumacetat, 3-Picoliniumacetat, 2,4-Lutidiniumacetat, 3,4-Lutidiniumacetat.

Die Promotoreigenschaft dieser Addukte wird durch die Anwesenheit einer aliphatischen Carbonsäure mit 1-8 Kohlenstoffatomen erheblich verstärkt.

Bevorzugt wird das Verfahren der Erfindung bei Temperaturen von 400-475 K und Drücken von 20-150 bar durchgeführt. Je Mol Methylacetat und/oder Dimethylether setzt man vorzugsweise 0,000 1-0,01 Mol des Edelmetalls der Gruppe VIII des Periodensystems der Elemente oder seiner Verbindungen ein. Vorzugsweise setzt man das Katalysatorsystem Edelmetall (-verbindung)/Mangan- oder Rheniumverbindung/Jod (-verbindung)/Carbonsäure/heterocyclische Verbindung im Atom- bzw. Molverhältnis 1 : (0,5-8) : (10-300) : (25-600) : (10-300) ein. Der Einsatz von Essigsäure als Carbonsäure wird bevorzugt.

Das erfindungsgemäße Verfahren, das vorzugsweise in flüssiger Phase abläuft, kann in einer einzigen oder einer Vielzahl hintereinander oder parallel geschalteter Reaktionszonen durchgeführt werden. Das Verfahren kann sowohl kontinuierlich als auch diskontinuierlich betrieben werden. Als Reaktionszone dienen ein oder mehrere Autoklaven oder eine oder mehrere längere, parallel geschaltete, röhrenförmige Reaktionsstrecken. Wandstärke und Material sind so gewählt, daß sie der Reaktionstemperatur und dem herrschenden Druck standhalten und gegenüber den Reaktionsmedien inert sind. Als Material eignen sich besonders Edelstähle vom Typ Hastelloy B und C sowie zirkonausgekleidete Reaktionsbehälter. Zur Abführung der Reaktionswärme ist die Reaktionszone mit inneren und/oder äußeren Wärmeaustauschern versehen, die die Aufrechterhaltung einer konstanten Reaktionstemperatur gewährleisten. Rührwerk oder Zwangsumlauf der flüssigen Reaktionsmedien garantieren eine gute Durchmischung der Gas- und Flüssigphase.

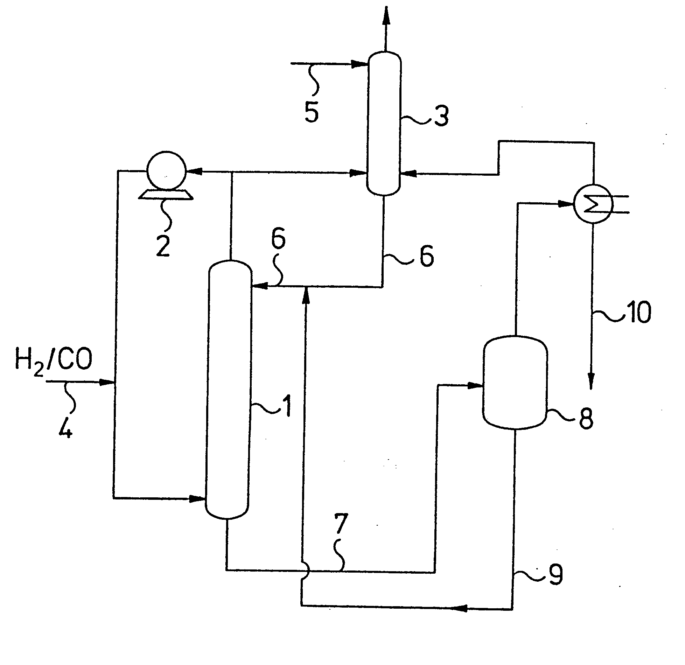

Ein mögliches Fließschema zur Durchführung des erfindungsgemäßen Verfahrens ist in der Zeichnung schematisch dargestellt und im folgenden beschrieben :

In einem Druckreaktor 1 aus Hastelloy C werden Methylacetat und/oder Dimethylether mit einem Gemisch aus Kohlenmonoxid und Wasserstoff in Gegenwart eines Katalysatorsystems aus Edelmetallen der Gruppe VIII des Periodensystems der Elemente oder deren Verbindungen und Jod und/oder dessen Verbindungen, vorzugsweise Methyljodid, unter Zusatz von Verbindungen des Mangans oder Rheniums sowie in Gegenwart einer Carbonsäure, vorzugsweise Essigsäure, und mindestens einer heterocyclischen aromatischen Verbindung, in der mindestens ein Heteroatom ein quaternäres Stickstoffatom ist, unter einem Druck von vorzugsweise 20-150 bar und einer Temperatur von vorzugsweise 400-475 K zu 1,1-Diacetoxiethan umgesetzt.

Nichtumgesetztes Kohlenmonoxid und Wasserstoff werden über eine Gasumwälzpumpe 2 im Kreislauf geführt, während ein geringer Teilstrom das System als Abgas über eine Wäsche 3 verläßt. Frisches Kohlenmonoxid und Wasserstoff werden dem Umsatz entsprechend über Leitung 4 dem Kreislaufgas zudosiert. Die dem Umsatz entsprechende Menge frischen Methylacetats und/oder Dimethylethers wird über Leitung 5 am Kopf der Wäsche 3 aufgegeben und über Leitung 5 am Kopf der Wäsche 3 aufgegeben und über Leitung 6 dem Reaktor 1 zugeleitet. Das Reaktionsgemisch strömt über Leitung 7 aus dem Reaktor 1 ab und wird im Verdampfer 8 von den nicht flüchtigen Bestandteilen des Katalysatorsystems abgetrennt, die über Leitung 9 und 6 zum Reaktor 1 zurückgeführt werden. Das über Leitung 10 erhaltene Kondensat wird in einer nicht zum erfindungsgemäßen Verfahren gehörenden Aufarbeitungsstufe destillativ getrennt. Nichtumgesetzte Ausgangsprodukte sowie der dem Gehalt im Katalysatorsystem entsprechende Anteil Essigsäure werden in die Reaktionszone 1 zurückgeführt. Die neben Essigsäure erhaltenen Reaktionsprodukte 1,1-Diacetoxiethan und Essigsäureanhydrid werden durch gaschromatographische Analyse sowie durch Kernresonanzanalyse bestimmt und durch abschließende fraktionierte Destillation in an sich bekannter Weise getrennt.

Das erfindungsgemäße Verfahren wird durch folgende Beispiele näher erläutert :

Beispiel 1 (nicht gemäß der Erfindung)

250 g Methylacetat, 1,6 g $RhCl_3 \cdot 3H_2O$, 50 g $CH_3J$, 25 g Essigsäure und 68 g N,N-Dimethylimidazoliumjodid werden in einem Hastelloy-Autoklaven mit einem Gemisch aus 60 Vol % CO und 40 Vol % $H_2$ unter einem Gesamtdruck von 100 bar bei 435 K umgesetzt. Nach einer Reaktionszeit von 23 min werden aus dem Reaktionsgemisch 109 g eines Gemisches mit 60 Masse % 1,1-Diacetoxiethan und 40 Masse % Essigsäureanhydrid erhalten. Dies entspricht einer Katalysatorleistung von 271 g 1,1-Diacetoxiethan/g Rh · h und 183 g Essigsäureanhydrid/g Rh · h.

Beispiel 2 (nicht gemäß der Erfindung)

250 g Methylacetat, 1,6 g $RhCl_3 \cdot 3H_2O$, 5,4 g $Cr(CO)_6$, 50 g $CH^3J$, 25 g Essigsäure und 68 g N,N-Dimethylimidazoliumjodid werden in einem Hastelloy-Autoklaven mit einem Gemisch aus 60 Vol % CO und 40 Vol % $H_2$ unter einem Gesamtdruck von 100 bar bei 435 K umgesetzt. Nach einer Reaktionszeit von 20 min werden als Reaktionsprodukt 146 g eines Gemisches mit 37,7 Masse % 1,1-Diacetoxiethan und 62,3 Masse % Essigsäureanhydrid erhalten. Hieraus ergibt sich eine Katalysatorleistung von 264 g 1,1-Diacetoxiethan/g Rh · h und 436 g Essigsäureanhydrid/g Rh · h.

Beispiel 3

250 g Methylacetat, 1,6 g Rh Cl$_3$ · 3H$_2$O, 4 g Mn (OAc)$_2$ · 4H$_2$O, 50 g CH$_3$J, 25 g Essigsäure und 68 g N,N-Dimethylimidazoliumjodid werden in einem Hastelloy-Autoklaven mit einem Gemisch aus 60 Vol % CO und 40 Vol % H$_2$ unter einem Gesamtdruck von 100 bar bei 435 K umgesetzt. Nach einer Reaktionszeit von 20 min werden aus dem Reaktionsprodukt 141 g eines Gemisches mit 58,7 Masse % 1,1-Diacetoxiethan und 41,3 Masse % Essigsäureanhydrid isoliert. Die entspricht einer Katalysatorleistung von 397 g 1,1-Diacetoxiethan/g Rh · h und 279 g Essigsäureanhydrid/g Rh · h.

Beispiel 4

250 g Methylacetat, 1,6 g RhCl$_3$ · 3H$_2$O, 3,9 g Re$_2$(CO)$_{10}$, 50 g CH$_3$J, 25 g Essigsäure und 68 g N,N-Dimethylimidazoliumjodid werden in einem Hastelloy-Autoklaven mit einem Gemisch aus 60 Vol % CO und 40 Vol % H$_2$ unter einem Gesamtdruck von 100 bar bei 435 K umgesetzt. Nach einer Reaktionszeit von 17 min werden aus dem Reaktionsprodukt 145 g eines Gemisches mit 60,9 Masse % 1,1-Diacetoxiethan und 39,1 Masse % Essigsäureanhydrid erhalten. Daraus ergibt sich eine Katalysatorleistung von 498 g 1,1-Diacetoxiethan/g Rh · h und 320 g Essigsäureanhydrid/g Rh · h.

Beispiel 5

250 g Methylacetat, 1 g RhCl$_3$ · 3H$_2$O, 1 g RuCl$_3$ · 3H$_2$O, 4 g Mn(OAc)$_2$ · 4H$_2$O, 70 g CH$_3$J, 30 g Essigsäure und 80 g N,N-Dimethylimidazoliumjodid werden in einem Hastelloy-Autoklaven mit einem Gemisch aus 60 Vol % CO und 40 Vol % H$_2$ unter einem Druck von 80 bar bei 440 K umgesetzt. Nach einer Reaktionszeit von 20 min werden aus dem Reaktionsprodukt 151 g eines Gemisches aus 62,8 Masse % 1,1-Diacetoxiethan und 37,2 Masse % Essigsäureanhydrid isoliert. Dies entspricht einer Katalysatorleistung von 728 g 1,1-Diacetoxiethan/g Rh · h und 431 g Ac$_2$O/g Rh · h.

Beispiel 6

250 g Methylacetat, 2,5 g (NH$_4$)$_2$IrCl$_6$, 4 g Mn(OAc)$_2$ · 4H$_2$O, 60 g CH$_3$J, 30 g Essigsäure und 60 g N,N-Dimethylimidazoliumjodid werden in einem Hastelloy-Autoklaven mit einem Gemisch aus 60 Vol % CO und 40 Vol % H$_2$ unter einem Druck von 80 bar bei 450 K umgesetzt. Nach einer Reaktionszeit von 26 min werden aus dem Reaktionsprodukt 139 g eines Gemisches aus 59,3 Masse % 1,1-Diacetoxiethan und 40,7 Masse % Essigsäureanhydrid isoliert. Daraus wird eine Katalysatorleistung von 175 g 1,1-Diacetoxiethan/g Ir · h und 120 g Ac$_2$O/g Ir · h ermittelt.

Beispiel 7

250 g Dimethylether, 1,8 g RhCl$_3$ · 3H$_2$O, 6 g Mn(OAc)$_2$ · 4H$_2$O, 70 g CH$_3$J, 80 g Essigsäure und 70 g N,N-Dimethylimidazoliumjodid werden in einem Hastelloy-Autoklaven mit 60 Vol % CO und 40 Vol % H$_2$ unter einem Druck von 80 bar bei 440 K umgesetzt. Nach einer Reaktionszeit von 16 min werden aus dem Reaktionsprodukt 149 g eines Gemisches aus 58,1 Masse % 1,1-Diacetoxiethan und 41,9 Masse % Essigsäureanhydrid abgetrennt. Daraus wird eine Katalysatorleistung von 461 g 1,1-Diacetoxiethan/g Rh · h und 333 g Ac$_2$O/g Rh · h errechnet.

Beispiel 8

250 g Methylacetat, 1,8 g RhCl$_3$ · 3H$_2$O, 6 g Mn(OAc)$_2$ · 4H$_2$O, 70 g CH$_3$J, 30 g Essigsäure, 60 g Essigsäureanhydrid und 100 g N,N-Dimethylimidazoliumjodid werden in einem Hastelloy-Autoklaven mit einem Gemisch aus 60 Vol % CO und 40 Vol % H$_2$ unter einem Druck von 80 bar bei 440 K umgesetzt. Nach einer Reaktionszeit von 19 min werden aus dem Reaktionsprodukt 241 g eines Gemisches aus 67,2 Masse % 1,1-Diacetoxiethan und 32,8 Masse % Essigsäureanhydrid isoliert. Hieraus errechnet sich eine Katalysatorleistung von 727 g 1,1-Diacetoxiethan/g Rh · h und 85,5 g Ac$_2$O/g Rh · h.

Beispiel 9

250 g Methylacetat, 0,8 g RhCl$_3$ · 3H$_2$O, 5 g MnCl$_2$ · 4H$_2$O, 80 g CH$_3$J, 40 g Essigsäure und 120 g N-Methyl-3-picoliniumjodid werden in einem Hastelloy-Autoklaven mit einem Gemisch aus 60 Vol % CO und 40 Vol % H$_2$ unter einem Druck von 70 bar bei 440 K umgesetzt. Nach einer Reaktionszeit von 21 min werden aus dem Reaktionsprodukt 153 g eines Gemisches aus 63,1 Masse % 1,1-Diacetoxiethan und 38,7 Masse % Essigsäureanhydrid isoliert. Dies entspricht einer Katalysatorleistung von 857 g 1,1-Diacetoxiethan/g Rh · h und 541 g Ac$_2$O/g Rh · h.

Beispiel 10

250 g Methylacetat, 1,6 g RhCl$_3$ · 3H$_2$O, 10 g Re$_2$(CO)$_{10}$, 90 g CH$_3$J, 30 g Essigsäure und 80 g 3-Picoliniumacetat werden in einem Hastelloy-Autoklaven mit einem Gemisch aus 60 Vol % CO und 40 Vol % H$_2$ unter einem Druck von 80 bar bei 435 K umgesetzt. Nach einer Reaktionszeit von 17 min werden aus dem Reaktionsprodukt 158 g eines Gemisches aus 63,2 Masse % 1,1-Diacetoxiethan und 36,8 Masse % Essigsäureanhydrid isoliert. Dies ergibt eine Katalysatorleistung von 564 g 1,1-Diacetoxiethan/g Rh · h und 328 g Ac$_2$O/g Rh · h.

Beispiel 11

250 g Methylacetat, 2,5 g Pd(OAc)$_2$, 8 g

$Re_2(CO)_{10}$, 90 g $CH_3J$, 30 g Essigsäure und 60 g N,N-Dimethylimidazoliumjodid werden in einem Hastelloy-Autoklaven mit einem Gemisch aus 60 Vol % CO und 40 Vol % $H_2$ unter einem Druck von 80 bar bei 450 K umgesetzt. Nach einer Reaktionszeit von 21 min werden aus dem Reaktionsprodukt 151 g eines Gemisches aus 89,3 Masse % 1,1-Diacetoxiethan und 10,7 Masse % Essigsäureanhydrid isoliert. Hieraus errechnet sich eine Katalysatorleistung von 325 g 1,1-Diacetoxiethan/g Pd · h und 39 g $Ac_2O$/g Pd · h.

**Ansprüche**

1. Verfahren zur Herstellung von 1,1-Diacetoxiethan durch Umsetzung von Methylacetat und/oder Dimethylether mit Kohlenmonoxid und Wasserstoff unter praktisch wasserfreien Bedingungen bei Temperaturen von 350 bis 575 K und Drücken von 1 bis 300 bar in Gegenwart eines Katalysatorsystems, welches Edelmetalle der Gruppe VIII des Periodensystems der Elemente oder deren Verbindungen und Jod und/oder dessen Verbindungen enthält, dadurch gekennzeichnet, daß man in Gegenwart eines Katalysatorsystems arbeitet, welches zusätzlich eine aliphatische Carbonsäure mit 1-8 Kohlenstoffatomen, eine heterocyclische aromatische Verbindung, in der mindestens ein Heteroatom ein quaternäres Stickstoffatom ist und deren Schmelz- oder Mischschmelzpunkt unterhalb 410 K liegt, sowie eine Verbindung des Mangans oder Rheniums enthält.

2. Verfahren nach Anspruch 1, dadurch gekennzeichnet, daß man die heterocyclischen Verbindungen in Form ihrer Addukte mit Essigsäure oder Methyljodid einsetzt.

3. Verfahren nach einem der vorhergehenden Ansprüche, dadurch gekennzeichnet, daß man das Katalysatorsystem Edelmetall (-verbindung)/Mangan- oder Rheniumverbindung/Jod (-verbindung)/Carbonsäure/heterocyclische Verbindung im Atom- bzw. Molverhältnis 1 : (0,1-10) : (1-1 400) : (10-2 000) : (1-1 200) einsetzt.

4. Verfahren nach einem der vorhergehenden Ansprüche, dadurch gekennzeichnet, daß man Kohlenmonoxid/Wasserstoffgemische mit 25 bis 75 Volum % Wasserstoff einsetzt.

**Claims**

1. Process for making 1,1-diacetoxiethane by reacting methyl acetate and/or dimethylether with carbon monoxide and hydrogen under practically anhydrous conditions, at temperatures of 350 to 575 K, under pressures of 1 to 300 bars, in the presence of a catalyst system containing noble metals belonging to group VIII of the periodic system of the elements, or their compounds, and iodine and/or its compounds, which comprises : effecting the reaction in the presence of a catalyst system containing, as additional ingredients, an aliphatic carboxylic acid having 1 to 8 carbon atoms, a heterocyclic aromatic compound, in which at least one heteroatom is a quaternary nitrogen atom, and which has a melting point or mixed melting point of less than 410 K, as well as a manganese or rhenium compound.

2. Process as claimed in claim 1, wherein the heterocyclic compounds are used in the form of their addition products with acetic acid or methyl iodide.

3. Process as claimed in one of the preceding claims, wherein the catalyst system comprised of noble metal (compound)/manganese or rhenium compound/iodine (compound)/carboxylic acid/heterocyclic compound is used in an atomic or molar ratio of 1 : (0.1-10) : (1-1 400) : (10-2 000) : (1-1 200).

4. Process as claimed in one of the preceding claims, wherein a carbon monoxide/hydrogenmixture containing 25 to 75 volume % hydrogen is used.

**Revendications**

1. Procédé de préparation de 1,1-diacétoxyéthane par réaction d'acétate de méthyle et/ou d'éther diméthylique avec le monoxyde de carbone et l'hydrogène dans des conditions pratiquement anhydres à des températures de 350 à 575 K et sous des pressions de 1 à 300 bars en présence d'un système catalytique contenant des métaux nobles appartenant au groupe VIII de la Classification Périodique des Eléments et/ou leurs composés et de l'iode et/ou ses composés, caractérisé en ce que l'on opère en présence d'un système catalytique contenant, comme constituants supplémentaires, un acide carboxylique aliphatique en $C_1$-$C_8$, un composé aromatique hétérocyclique, dans lequel au moins un hétéroatome est un atome d'azote quaternaire et dont le point de fusion ou point de fusion à l'épreuve de mélange est inférieur à 410 K, ainsi qu'un composé de manganèse ou de rhénium.

2. Procédé selon la revendication 1, caractérisé en ce que l'on utilise les composés hétérocycliques sous forme de leurs produits d'addition avec l'acide acétique ou l'iodure de méthyle.

3. Procédé selon l'une des revendications précédentes, caractérisé en ce que l'on utilise le système catalytique (composé) de métal noble/composé de manganèse ou de rhénium/(composé) d'iode/acide carboxylique/composé hétérocyclique dans un rapport atomique ou molaire de 1 : (0,1-10) : (1-1 400) : (10-2 000) : (1-1 200).

4. Procédé selon l'une des revendications précédentes, caractérisé en ce que l'on utilise des mélanges monoxyde de carbone/hydrogène à 25-75 % en volume d'hydrogène.